# EUROPEAN PATENT SPECIFICATION

(11) **EP 1 032 605 B1**
(45) Date of publication and mention of the grant of the patent: **14.11.2007**
(21) Application number: 98946004.3
(22) Date of filing: 10.09.1998
(51) Int. Cl.: C08G 63/00, C08G 63/02, C08G 67/00, C08G 69/00

(54) **POLYANHYDRIDES WITH THERAPEUTICALLY USEFUL DEGRADATION PRODUCTS**
POLYAMHYDRIDE MIT THERAPEUTISCH VERWENDBAREN ABBAUPRODUKTEN
POLYANHYDRES AVEC PRODUITS DE DEGRADATION UTILISES THERAPEUTIQUEMENT

(30) Priority: 10.09.1997 US 58328 P
(43) Date of publication of application: 06.09.2000
(60) Divisional application: 07004364.1
(73) Proprietor: Rutgers, The State University, New Brunswick, New Jersey 08903 (US)
(72) Inventor: UHRICH, Kathryn, Hoboken, NJ 07030 (US)
(74) Representative: Hammer, Jens
(86) International application number: PCT/US1998/018816
(87) International publication number: WO 1999/012990

(56) References cited:
- DD-A- 288 311
- DD-A- 288 387
- US-A- 4 792 598
- US-A- 4 997 904
- US-A- 5 264 540
- US-A- 5 364 725
- ERDMANN LAURA ET AL: "Polymer prodrugs with pharmaceutically active degradation products" PROCEEDINGS OF THE 1997 LAS VEGAS ACS MEETING;LAS VEGAS, NV, USA SEP 7-12 1997, vol. 38, no. 2, September 1997 (1997-09), pages 570-571, XP002184405 Polym Prepr Div Polym Chem Am Chem Soc;Polymer Preprints, Division of Polymer Chemistry, American Chemical Society Sept 1997 ACS, Washington, DC, USA
- ERDMANN LAURA ET AL: "Polymeric salicylic acid: in vitro and in vivo degradation" PROCEEDINGS OF THE 1997 BOSTON MEETING;BOSTON, MA, USA AUG 23-27 1998, vol. 39, no. 2, August 1998 (1998-08), pages 224-225, XP002184406 Polym Prepr Div Polym Chem Am Chem Soc;Polymer Preprints, Division of Polymer Chemistry, American Chemical Society Aug 1998 ACS, Washington, DC, USA
- PINTHER P ET AL: "SYNTHESIS OF POLYANHYDRIDES CONTAINING ESTER GROUPS" MAKROMOLEKULARE CHEMIE, RAPID COMMUNICATIONS, HUTHIG UND WEPF VERLAG. BASEL, CH, vol. 11, no. 8, 1 August 1990 (1990-08-01), pages 403-408, XP000165931
- DATABASE BIOSIS [Online] BIOSCIENCES INFORMATION SERVICE, PHILADELPHIA, PA, US; GIAMMONA G. & AL: "Polymeric Prodrugs alpha beta poly-hydroxyethyl-dl-aspartamide as macromolecular carrier for some non-steroidal anti-inflammatory agents" XP002184408
- DOMB A J: "SYNTHESIS AND CHARACTERIZATION OF BIODEGRADABLE AROMATIC ANHYDRIDE COPOLYMERS" MACROMOLECULES, AMERICAN CHEMICAL SOCIETY. EASTON, US, vol. 25, no. 1, 6 January 1992 (1992-01-06), pages 12-17, XP000259166 ISSN: 0024-9297
- CHAFI N., MONTHEARD J.P. AND VERGNAUD J.M.: "Dosage form with salicylic acid attached to a polyanhydride polymer dispersed in a Eudragit matrix" INTERNATIONAL JOURNAL OF PHARMACEUTICS, vol. 52, 1989, pages 203-211, XP001037634
- CHATTERJEE R., WALDER Y.W., ARNONE A. AND WALDER J.A.: "Mechanism for the increase in solubility of Deoxyhemoglobin S due to Cross-linking the b Chains between Lysine-82b1 and Lysine-82b2" BIOCHEMISTRY, vol. 21, 1982, pages 5901-5909, XP001036952

## Description

The present invention relates to biocompatible aromatic polyanhydrides having improved degradation properties and processability and unique therapeutic properties. In particular, the present invention relates to aromatic polyanhydrides produced from ortho-substituted bis-aromatic carboxylic acid anhydrides. The present invention also relates to ortho-substituted bis-aromatic dicarboxylic acids useful in the preparation of the aromatic polyanhydrides of the present invention.

### BACKGROUND ART

Biocompatible and biodegradable aromatic polyanhydrides are disclosed by U.S. Patent Nos. 4,757,128 and 4,997,904. However, unless incorporated into a copolymer containing a more hydrophilic monomer, such as sebacic acid, the aromatic polyanhydrides of the prior art have slow degradation times as well as relatively insoluble degradation products. A major drawback to the prior art aromatic polyanhydrides is their insolubility in most organic solvents.

Biocompatible and biodegradable aromatic polyanhydrides prepared from para-substituted bis-aromatic dicarboxylic acids are disclosed by U.S. Patent No. 5,264,540. The para-substitution pattern results in higher melt and glass transition temperatures and decreased solubility, thus ultimately making these para-substituted polymers difficult to process.

DD 288311 A5 discloses para-substituted polyanhydrides.

A need exists for biocompatible and biodegradable aromatic polyanhydrides having improved degradation and processing properties, as well as therapeutic utilities.

### SUMMARY OF THE INVENTION

This need is met by the present invention. It has now been discovered that the preparation of aromatic polyanhydrides from ortho-substituted bis-aromatic carboxylic acid anhydrides disrupts the crystallinity of the resulting polymer, enhancing solubility and processability, as well as degradation properties. Therefore, according to one aspect of the present invention, an aromatic polyanhydride is provided having a repeated unit within the structure of Formula I: 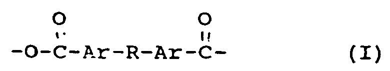 as defined in claim 1 and claim 2. Further aspects of the present invention are defined in claims 3 to 40. Ar and R are preferably selected so that the hydrolysis products of the polyanhydrides have a chemical structure resembling pharmaceutically-active materials, particularly salicyclates such as aspirin, non-steroidal anti-inflammatory naphthyl or phenyl propionates such as ibuprofen, ketoprofen, naproxen, and the like, or other aromatic anti-inflammatory compounds such as indomethacin, indoprofen, and the like. In particular, Ar is preferably a phenyl group and R is preferably -Z₁-R₁-Z₁₋ in which R₁ is a difunctional moiety and both Z₁'s are independently either an amide, anhydride, carbonate, or urethane groups. R₁ is preferably an alkylene group containing from 1 to 20 carbon atoms, or a group with 2-20 carbon atoms having a structure selected from (-CH₂-CH₂-O-)ₘ, (-CH₂-CH₂-CH₂-O-)ₘ, and (-CH₂-CHCH₃-O-)ₘ.

Ortho-substituted bis-aromatic carboxylic acid anhydrides are intermediate compounds having utility in the preparation of the aromatic polyanhydrides of the present invention. Such intermediates are in particular ortho-substituted bis-aromatic carboxylic acid anhydrides having the structure of Formula II: 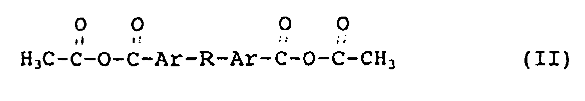 wherein Ar and R, and the preferred species thereof, are the same as described above with respect to Formula I and R is substituted on each Ar ortho to the anhydride group.

Other suitable intermediates are ortho-substituted bis-aromatic dicarboxylic acids having the structure of HOOC-Ar-R-Ar-COOH, wherein Ar and R, and the preferred species thereof, are the same as described above with respect to Formula I, and R is substituted on each Ar ortho to each carboxylic acid group.

The aromatic polyanhydrides of the present invention meet the need for moldable biocompatible biodegradable polymers. Therefore, the present invention also includes implantable medical devices as defined in the claims containing the aromatic polyanhydrides of the present invention. When Ar and R are selected so that the aromatic polyanhydride hydrolyzes to form therapeutic salicyclates, the aromatic polyanhydrides have potential uses as biocompatible, biodegradable scaffolding implants for tissue reconstruction in which the degradation products have anti-thrombogenic qualities.

In addition, the aromatic polyanhydrides that hydrolyze to form therapeutic salicyclates have potential uses as anti-inflammatory dosage forms, including dosage forms for oral administration, particularly in the treatment of digestive disorders, including bowel disorders such as inflammatory bowel disease, Crohn's disease, and the like. Ar and R may also be selected so that the aromatic polyanhydrides hydrolyze to form therapeutic non-steroidal anti-inflammatory naphthyl and phenyl propionates that resemble compounds such as ibuprofen, ketoprofen, naproxen, and the like, and other aromatic anti-inflammatory compounds such as indomethacin, indoprofen, and the like.

Therefore, the present invention also includes a method for treating inflammation by administering to a patient in need thereof a quantity of the aromatic polyanhydride of the present invention in which Ar and R are selected so that aromatic polyanhydride hydrolyzes to form therapeutic salicyclates at the site of inflammation in an amount effective to relieve the inflammation. The aromatic polyanhydrides may be administered orally. This is particularly useful in the treatment of digestive inflammation, such as inflammatory bowel disease, because the therapeutic salicyclates are formed in the gastro-intestinal tract of the patient. Methods for treating inflammation with aromatic polyanhydrides that hydrolyze to form therapeutic naphthyl or phenyl propionates are included in the present invention as well, as well as methods for treating inflammtion with aromatic polyanhydrides that hydrolyze to form indomethacin or indoprofen.

The present invention therefore also includes anti-inflammatory oral dosage forms consisting essentially of the aromatic polyanhydrides of the present invention that hydrolyze to form therapeutic salicyclates or naphthyl or phenyl propionates, or indomethacin or indoprofen, and a pharmaceutically acceptable excipient. The oral dosage forms may further include a biologically or pharmaceutically active compound to be co-administered with the therapeutic degradation products.

Ar and R may also be selected so that the aromatic polyanhydrides hydrolyzes to form therapeutic antiulcerative drugs such as rosaprostol, therapeutic antifibrotic aminobenzoates and therapeutic vasoconstricting phenylethanolamines and vasoconstricting drugs such as midodrine. Therefore, the present invention also includes a method for therapeutic treatment by administering to a patient in need thereof a quantity of the aromatic polyanhydride of the present invention in which Ar and R are selected so that aromatic polyanhydride hydrolyzes to form rosaprostol, antifibrotic aminobenzoates, vasoconstricting phenylethanolamines and midodrine. The present invention also includes oral dosage forms consisting essentially of the aromatic polyanhydrides of the present invention in which Ar and R are selected so that the aromatic polyanhydrides hydrolyze to form rosaprostol, antifibrotic aminobenzoates, vasoconstricting phenylethanolamines and midodrine.

In another embodiment of the present invention, the aromatic polyanhydrides are combined with a quantity of biologically or pharmaceutically active compound sufficient for effective site-specific or systemic drug delivery as described by Gutkowsky et al., J. Biomater. Res., 29, 811-21 (1995) and Hoffman, J. Controlled Release, 6, 297-305 (1987). The biologically or pharmaceutically active compound may be physically admixed, embedded or dispersed in the polymer matrix. Alternatively, derivatives of biologically and pharmaceutically active compounds can be attached to repeating units of the polymers of the present invention by covalent bonds linked to an Ar ring or an R organic moiety. This provides for sustained release of the biologically or pharmaceutically active compound.

Another aspect of the present invention provides a method for site-specific or systemic drug delivery by implanting in the body of a patient in need thereof an implantable drug delivery device containing a therapeutically effective amount of a biologically or pharmaceutically active compound in combination with an aromatic polyanhydride of the present invention.

A more complete appreciation of the invention and many more other intended advantages can be readily obtained by reference to the following detailed description of the preferred embodiments and claims, which disclose the principles of the invention and the best modes which are presently contemplated for carrying them out.

### DETAILED DESCRIPTION OF THE PREFERRED EMBODIMENTS

The present invention provides aromatic polyanhydrides with improved degradation properties and processability having repeating units with the structure of Formula I in which Ar and R are the same as described above with respect to Formula I. R preferably has a structure of -Z₁-R₁-Z₁-, in which R₁ is a difunctional organic moiety and both Z₁'s are difunctional moieties independently selected from amides, anhydrides, urethanes, carbamates, or carbonates, R₁ may be an alkylene group containing from 1 to 20, and preferably 6, carbon atoms, or R₁ may be a group having from 2 to 30, and preferably 6, carbon atoms having a structure selected from (-CH₂-CH₂-O-)ₘ, (-CH₂-CH₂-CH₂-O-)ₘ,and (-CH₂-CHCH₃-O-)ₘ, or R₁ may have the structure -R₂-Z₂-R₃-, wherein R₂ and R₃ are independently alkylene groups containing from 1 to 19 carbon atoms or groups having from 2 to 18 carbon atoms having a structure selected from (-CH₂-CH₂-O-)ₘ, (-CH₂-CH₂-CH₂-O-)ₘ, and (-CH₂-CHCH)-O-)ₘ, and Z₂ is selected from the difunctional moieties described above with respect to Z₁.

Ar may be an alkylaryl group, in which a difunctional organic moiety is positioned between each anhydride carbonyl group and the corresponding aromatic ring. Preferably, however, each carbonyl group is directly substituted on the corresponding aromatic ring.

Preferred polymers of the present invention have repeating units with the structure of Formula I in which Ar is a phenyl ring and R is selected from -Z₁-(-CH₂-)ₙ-Z₁-, -Z(-CH₂-CH₂-O-)ₘ-Z₁-, -Z(-CH₂-CH₂-CH₂-O-)ₘ-Z₁-, and -Z(-CH₂-CHCH₃-O-)ₘ-Z₁-, wherein Z₁ is an amide group and n is from 1 to 20 inclusive, and preferably is 6, and m is selected so that R has from 2 to 20, and preferably 6, carbon atoms.

The aromatic polyanhydrides of the present invention may be prepared by the method described in Conix, Macromol. Synth., 2, 95-99 (1996), in which dicarboxylic acids are acetylated in an excess of acetic anhydride at reflux temperatures followed by melt condensation of the resulting carboxylic acid anhydride at 180°C for 2-3 hours. The resulting polymers are isolated by precipitation into diethyl ether from methylene chloride. The described process is essentially the conventional method for polymerizing bis-aromatic dicarboxylic acid anhydrides into aromatic polyanhydrides.

Aromatic polyanhydrides in accordance with the present invention have weight average molecular weights of at least about 1500 daltons, up to about 35,000 daltons, calculated by Gel Permeation Chromatography (GPC) relative to narrow molecular weight polystyrene standards.

The aromatic polyanhydrides of the present invention are produced from orth-substituted bis-aromatic carboxylic acid anhydrides having the structure of Formula II in which Ar, R and the preferred species thereof are the same as described above with respect to Formula I. As noted above, ortho-substituted bis-aromatic carboxylic acid anhydrides are prepared by acetylation of the corresponding ortho-substituted bis-aromatic carboxylic acids in an excess of acetic anhydride at reflux temperatures. The dicarboxylic acids have the structure of Formula III, wherein Ar, R and the preferred species thereof are the same as described above with respect to Formula I.

The dicarboxylic acids are prepared by reacting a stiochiometric ratio of aromatic carboxylic acid having the structure Z₃-Ar-COOH and a compound having a structure Z₄-R-Z₂ wherein Ar is a substituted or unsubstituted aromatic ring on which Z₃ is substituted ortho to the carboxylic acid group, R is a difunctional organic moiety and Z₂ and Z₄ are functional groups selected to provide the linkage desired between the difunctional organic moiety and the two aromatic rings.

Suitable Z₃ and Z₄ functional groups, and the manner in which they may be reacted to produce the bis-aromatic dicarboxylic acids of the present invention, may be readily determined by those of ordinary skill in the art without undue experimentation. For example, for aromatic polyanhydrides having the structure of Formula I in which Ar is a phenyl group and R is -O-(CH₂-)₆-O-, the ortho-substituted bis-aromatic dicarboxylic acid starting material may be prepared by reacting o-salicylic acid with 1,6-dibromohexane.

The aromatic polyanhydrides of the present invention can be isolated by known methods commonly employed in the field of synthetic polymers to produce a variety of useful articles with valuable physical and chemical properties. The new polymers can be readily processed by solvent casting to yield films, coatings, dishes and sponges with different geometric shapes for design of various medical implants, and may also be processed by compression molding and extrusion. Medical implant applications include the use of aromatic polyanhydrides to form shaped articles such as vascular graphs and stents, bone plates, sutures, implantable sensors, implantable drug delivery devices, stents for tissue regeneration, and other articles that decompose harmlessly within a known time period.

The polymers of the present invention include aromatic polyanhydrides having a repeating unit with the structure of Formula I in which Ar and R are selected to provide aromatic polyanhydrides that hydrolyze to form therapeutically useful salicyclates. As noted above, the salicyclates may be employed to treat inflammation, particularly digestive inflammation such as inflammatory bowel disorders. Thus, implantable or ingestible drug delivery devices of the present invention include oral dosage forms consisting essentially of the aromatic polyanhydrides of the present invention that hydrolyze to form therapeutic salicyclates, in combination with a pharmaceutically acceptable excipient. The oral dosage forms function to deliver salicyclates to the site of inflammation, either directly, or by being absorbed into the bloodstream from the digestive tract. The salicyclates may be supplemented with other therapeutic agents in the polymer matrix.

Examples of the therapeutic salicyclates include, but are not limited to, thymotic acid, 4, 4-sulfinyldinailine, 4-sulfanilamidosalicylic acid, sulfanilic acid, sulfanilylbenzylamine, sulfaloxic acid, succisulfone, salicylsulfuric acid, salsallate, salicylic alcohol, orthocaine, mesalamine, gentisic acid, enfenamic acid, cresotic acid, aminosalicylic acid, aminophenylacetic acid, acetylsalicylic acid. The identification of Ar and R moieties that provide aromatic polyanhydrides that hydrolyze to form such therapeutically useful salicyclates can be readily determined by those of ordinary skill in the art without undue experimentation.

Ar and R may also be selected so that the aromatic polyanhydrides hydrolyze to form therapeutic non-steroidal anti-inflammatory phenyl and naphthyl propionates, indomethacin and indoprofen. The identification of Ar and R moieties that provide aromatic polyanhydrides that hydrolyze to form such therapeutic anti-inflammatory compounds can also be readily determined by those of ordinary skill in the art without undue experimentation.

Ar and R may also be selected so that the aromatic polyanhydrides hydrolyze to form other therapeutic compounds. For example, Ar and R may be selected to provide an aromatic polyanhydride that hydrolyzes to form the antiulcerative drug rosaprostol. Ar and R may also be selected to provide aromatic polyanhydrides that hydrolyze to form antifibrotic aminobenzoates. Ar and R may further be selected to provide polyanhydrides that hydrolyze to form the vasoconstricting drug midodrine, as well as vasoconstricting phenylethanolamines. Again, the identification of Ar and R moieties that provide aromatic polyanhydrides that hydrolyze to form such therapeutic compounds can readily be determined by those of ordinary skill in the art without undue experimentation.

Pharmaceutically acceptable excipients for oral administration are well known and include diluents such as lactose, sucrose, mannitol, sorbitol, cellulose, glycine, and the like, lubricants such as silica, talc, stearic acid and salts thereof, binders such as magnesium aluminum silicate, starches such as corn starch, methyl cellulose, and disintegrating agents such as starches, agar, as well as dyestuffs, flavors and sweeteners. The dosage forms are manufactured in a manner that is in itself well known, for example, by means of conventional mixing, granulating or dragee-making processes.

The quantity of aromatic polyanhydride that hydrolyzes to form an amount of therapeutic salicyclate effective to relieve inflammation can be readily determined by those of ordinary skill in the art without undue experimentation. The quantity essentially corresponds stiochiometrically to the amount of salicyclate known to produce an effective treatment. Oral dosage forms of aromatic polyanhydrides that hydrolyze to form other therapeutic non-steroidal anti-inflammatory compounds and other therapeutic compounds are prepared and administered in a similar manner.

The ortho-substituted aromatic polyanhydrides of the present invention exhibit desirable adhesion to cell cultures. The disruption of crystallinity is believed to improve the attachment and growth of cells and may facilitate specific interactions with proteins, peptides and cells. The aromatic polyanhydrides of the present invention are thus useful as scaffolding implants for tissue reconstruction. The polymer surfaces may also be modified by simple chemical protocols to attach specific peptides or to immobilize proteins to elicit selective cellular responses in tissue engineering applications or in implant design.

Controlled drug delivery systems may also be prepared, in which a biologically or pharmaceutically active agent is physically embedded or dispersed into the polymeric matrix, physically admixed with, or covalently bonded to the aromatic polyanhydride. Covalent bonding is accomplished by providing an aromatic polyanhydride having reactive functional groups on one or more Ar groups or R moieties and reacting the polyanhydride with a derivatized or underivatized biologically or pharmaceutically active compound capable of reacting with the functional group on the aromatic polyanhydride to form a covalent bond. Thus, biologically or pharmaceutically active compounds may be linked to aromatic polyanhydrides by means of ester groups, amide groups.

Examples of biologically or pharmaceutically active compounds suitable for the use in the present invention include acyclovir, cephradrine, malphalan, procaine, ephedrine, adriamicin, daunomycin, plumbagin, atropine, quinine, digoxin, quinidine, biologically active peptides, chlorin e₆, cephadrine, cephalothin, penicillin IV, nicotinic acid, chemodeoxycholic acid, chlorambucil. Biologically active compounds, for the purposes of the present invention, are additionally defined as including cell mediators, biologically active ligands, and the like. The compounds are covalently bonded to the aromatic polyanhydride by methods well understood by those of ordinary skill in the art. Drug delivery compounds may also be formed by physically blending the biologically or pharmaceutically active compound to be delivered with the aromatic polyanhydrides of the present invention using conventional techniques well-known to those of ordinary skill in the art.

The following non-limiting examples set forth hereinbelow illustrate certain aspects of the invention. All parts and percentages are by weight unless otherwise noted and all temperatures are in degrees Celsius. Except for acetic anhydride and ethyl ether (Fisher Scientific), all solvents and reagents were obtained from Aldrich Chemical. All solvents were HPLC grade. All other reagents were of analytical grade and were purified by distillation or recrystallization.

All compounds were characterized by a proton nuclear magnetic resonance (NMR) spectroscopy, infrared (IR) spectroscopy, gel permeation chromatography (GPC), high performance liquid chromatography (HPLC), differential scanning calorimetry (DSC), and thermal gravimetric analysis (TGA). Infrared spectroscopy was performed on an AT1 Mattson Genesis (M100) FTIR Spectrophotometer. Samples were prepared by solvent casting on NaCl plates. ¹H and ¹³C NMR spectroscopy was obtained on a Varian 200 MHz or Varian 400 MHz spectrometer in solutions of CDCl₃ or DMSO-d₆ with solvent as the internal reference.

GPC was performed on a Perkin-Elmer Advanced LC Sample Processor (ISS 200) with PE series 200 LC Pump and a PE Series LC Refractive Index Detector to determine molecular weight and polydispersity. The data analysis was carried out using Turbochrom 4 software on a DEC Celebris 466 computer. Samples were dissolved in tetrahydrofuran and eluted through a mixed bed column (PE PL gel, 5 µm mixed bed) at a flow rate of 0.5 mL/min. Samples (about 5 mg/mL) were dissolved into the tetrahydrofuran and filtered using 0.5 µm PTFE syringe filters prior to column injection. Molecular weights were determined relative to narrow molecular weight polystyrene standards (Polysciences, Inc.).

Thermal analysis was performed on a Perkin-Elmer system consisting of a TGA 7 thermal gravimetric analyzer equipped with PE AD-4 autobalance and Pyris 1 DSC analyzer. Pyris software was used to carry out data analysis on a DEC Venturis 5100 computer. For DSC, an average sample weight of 5-10 mg was heated at 10°c/min. at a 30 psi flow of N₂. For TGA, an average sample weight of 10 mg was heated at 20C°/min under a 8 psi flow of N₂. Sessile drop contact angle measurements were obtained with an NRL Goniometer (Rame-hart) using distilled water. Solutions of polymer in methylene chloride (10% wt/vol.) were spun-coated onto glass slips, at 5,000 rpm for 30 seconds.

### EXAMPLES

### Example I-Preparation of 1,6-Bis(o-Carboxyphenoxy) Hexane Dicarboxylic Acid

To a mixture of salicylic acid (77.12 g, 0.5580 mole) and distilled water (84mL) sodium hydroxide (44.71 g, 1.120 mole) was added. The reaction was brought to reflux temperature before 1,6-dibromohexane (45.21 g, 0.2790 mole) was added drop-wise. Reflux was continued for 23 hours after which additional sodium hydroxide (11.17 g, 0.2790 mole) was added. The mixture was refluxed for 16 more hours, cooled, filtered, and washed with methanol. The yield was 48.8%.

### Example II-Preparation of 1,6-Bis(o-Carboxyphenoxy) Hexane Monomer (o-CPH)

The dicarboxylic acid of Example I was acetylated in an excess of acidic anhydride at reflux temperature. The resulting monomer was precipitated from methylene chloride into an excess of diethyl ether. The yield was 66.8%.

### Example III-Preparation of Poly(1,6-Bis(o-Carboxyphenoxy) Hexane) (Poly(o-CPH))

The monomer of Example II was polymerized in a melt condensation performed at 180°C for 3 hours under vacuum in a reaction vessel with a side arm. The polymerization vessel was flushed with nitrogen at frequent intervals. The polymer was isolated by precipitation into diethyl ether from methylene chloride. The yield was quantitative.

All compounds were characterized by nuclear magnetic resonance spectroscopy, GPC, differential scanning calorimetry (DSC), thermal gravimetric analysis, contact angle measurements, UV spectroscopy, mass spectroscopy, elemental analysis and high pressure liquid chromatography (HPLC).

The o-CPH monomer was polymerized by melt polycondensation for 60 minutes at temperatures ranging from 100° to 300°C. Analysis of the resulting polymers by GPC indicated that the highest molecular weight, coupled with the lowest polydispersity index occurred at 260°C.

The poly(o-CPH) was generally soluble in methylene chloride and chloroform, while the poly(p-CPH) was not. The poly(o-CPH) was slightly soluble in tetrahydrofuran, acetone and ethyl acetate.

Disks of poly(o-CPH), poly(p-CPH) and, as a reference, poly(lactic acid glycolic acid) were prepared and placed in 0.1 phosphate buffer solution at 37°C for 4 weeks. The degradation media was replaced periodically. The degradation profile was linear up to three weeks time.

In currently used polyanhydride systems, the aromatic groups are para-substituted. This substitution pattern results in higher melt and glass transition temperatures and decreased solubility, thus ultimately making these para-substituted polymers difficult to process.

Poly(o-CPH), unlike poly(p-CPH), has both a lower melting point (65°C vs. 143°C) and glass transition temperature (35°C vs. 47°C). It is also possible to solution cast poly(o-CPH) using low-boiling solvents whereas poly(p-CPH) is relatively insoluble in most organic and aqueous solvents. This structural modification gives a polymer whose hydrolysis products are chemically similar to aspirin. Aspirin is an anti-inflammatory agent derived from salicylic acid, which is one of the reagents used to synthesize the inventive polyanhydrides. Therefore, the degradation products of this polymer may actually aid in patient recovery. Because of pliability and ease of processing, the aromatic polyanhydrides of the present invention have great potential as polymer scaffolds for wound healing.

### Example IV-Preparation of 1,3-bis(o-carboxyphenoxy)propane dicarboxylic acid

1,3-dibromopropane (14.7 mL, 0.145 mole) was added to a mixture of salicylic acid (40.0 g, 0.290 mole), distilled water (44 mL) and sodium hydroxide (23.2 g, 0-580 mole) using the method described in Example I. After 4 hours, additional sodium hydroxide (5.79 g, 0.145 mole) was added to the reaction mixture. Reflux was continued for another 4 hours, after which the mixture was cooled, filtered and washed using the methods described in Example I. The yield was 37.7%

### Example V-Preparation of poly(1,3-bis(o-carboxyphenoxy)propane)

The dicarboxylic acid of Example IV was acetylated using the methods of Example II. The acetylated dicaboxylic acid was then polymerized using the methods described in Example III. The resulting polymer had a M_{w} of 8,500 daltons and a polydispersity of 2.3.

Contact angle measurements on solvent-cast films demonstrated that the hexyl chain of the polymer of Example III increased the surface hydrophobicity relative to the shorter propyl chain of the polymer of Example V. A comparison of thermal characteristics emphasized the effects of lengthening the alkyl chain. In particular, the polymer of Example III has a T_{g} of 34°C and a T_{d} of 410°C, while the polymer of Example V had a T_{g} of 50°C and a T_{d} of 344°C. Thus, the hexyl chain decreased the glass transition temperature (T_{g}) relative to the propyl chain, reflecting the increased flexibility of the polymer chain. The opposite trend was observed for decomposition temperatures (T_{d}), with the longer alkyl chain increasing the T_{d}.

Optimum polycondensation conditions were determined for the polymer of Example III. Optimum conditions were defined as those that yielded a crude polymer with the highest molecular weight and highest T_{g}. Higher reaction temperatures decreased the M_{w} values (measured by GPC) with a concurrent increase in polydispersity. As expected for a condensation polymerization, longer reaction times yielded polymers with higher molecular weights. However, over longer reaction times, there appeared a subsequent decrease in T_{g}. Based on these results, the optimum conditions were defined as temperatures of 220°C for 150 minutes under a vacuum.

### Example VI-Preparation of 1,8-bis[o-(benzylcarboxy)carboxy phenyl] octane dicarboxylic acid ester

The initial synthesis of poly(anhydride-ester) dicarboxylic acid monomers was attempted using the same methodology used for the poly(anhydride-ether) dicarboxylic monomers of Example III. It was found, however, that the reactivity of the phenol was enhanced by benzylation of the carboxylic acid group. In addition, the solubility of benzyl salicylate in organic media increased the ability of the reaction to move forward.

Thus, benzyl salicylate (1.530 g, 6.720 mmole) and distilled tetrahydrofuran were combined under an inert atmosphere in a reaction flask. An ice-salt bath was placed under the reaction flask and the addition of 60% sodium hydride (0.4840 g, 12.10 mmole) followed. After one hour, sebacoyl chloride (0.7850 g, 3.280 mmole) was added drop-wise to the 0°C reaction mixture. After 30 minutes, the reaction mixture was vacuum filtered, the filtrate collected and the solvent removed to reveal to yield the free carboxylate as a white solid residue. Purification was performed using a chromatron with ethyl acetate/methylene chloride (20/80) as the solvent system. The yield was 43%.

### Example VII-Polymerization of Poly(1,8-bis(o-dicarboxyphenyl) octane)

To remove the benzyl protecting groups, the 1,8-bis[(benzylcarboxy)carboxyphenyl]octane dicarboxylic acid ester of Example VI (0.06000 g, 0.9620 mmole) was dissolved in methylene chloride in a reaction flask (60.00 mL). The catalyst Pd-C (10%, 1.200 g) was added to the reaction flask. After 30 minutes, the reaction was complete. The reaction mixture was filtered and the solvent removed to yield the free dicarboxylic acid as a white solid residue which was recrystallized using petroleum ether and methylene chloride. The yield was 45%.

The dicarboxylic acid was acetylated using the methods described in Example II and the acetylated dicarboxylic acid was then polymerized using the methods described in Example III. The resulting polymer had a M_{w} of 3,000 daltons and a polydispersity of 1.40.

Subsequent polymerizations yielded polymers with M_{w}'s ranging from 2,000 to 5,000 daltons with corresponding polydispersities of approximately 1.40.

The poly(anhydride esters) of Example VII were compression molded into circular discs and placed in phosphate buffered saline solution under acidic, neutral and basic conditions. Over the course of a three-week degradation study, the polymers in the acidic and neutral solutions showed no observable changes, whereas the polymer in the basic media showed significant morphological changes over time.

### Example VIII-Preparation of Poly[(1,8-bis(o-dicarboxyphenyl) octane)-(1,6-bis(p-carboxyphenoxy) hexane] copolymers

The 1,8-bis(o-dicarboxyphenyl) octane of Example II was copolymerized with 1,6-bis(p-carboxyphenoxy) hexane using the methods described in Example III. In an in vivo mouse study, each mouse was implanted with 2 polymers, the copolymer of Example VIII and poly(1,6-bis(p-carboxyphenoxy)hexane). Each polymer was compression molded for 1 to 5 minutes at 1 to 20 K psi depending on the thickness of polymer needed. The polymer was placed under the palatal gingival mucosa adjacent to the first maxillary molars. The mice were sacrificed at 1, 4 and 10 day intervals and demonstrated the biocompatibility an biodegradability in vivo of the polymers of the present invention, with salicylic acid being released upon degradation, via hydrolysis of the polymer backbone.

### STATEMENT OF INDUSTRIAL APPLICABLITY

The polymers of the present invention have a variety of pharmaceutical applications, particularly as anti-inflammatory compounds.

## Claims

1. An aromatic polyanhydride comprising at least one repeating unit of the chemical formula 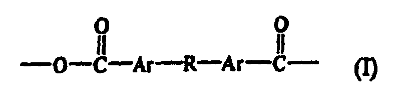 wherein
Ar is a substituted or unsubstituted aromatic ring and
R is -Z¹-R¹-Z¹- substituted ortho to the anhydride group on Ar, wherein R¹ is a difunctional organic moiety and Z¹ is a difunctional moiety selected from amide, anhydride, urethane, carbamate or carbonate.

2. An aromatic polyanhydride comprising at least one repeating unit of the chemical formula 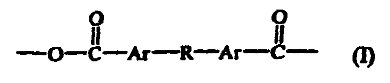 wherein
Ar is a substituted or unsubstituted aromatic ring and
R is - Z¹- R¹- Z¹- substituted ortho to the anhydride group on Ar, wherein R¹ is a group with 2 to 20 carbon atoms having a structure selected from (-CH₂CH₂-O-)ₘ, (-CN₂CH₂CH₂-O-)ₘ, and (-CH₂CHCH₃-O-)ₘ and Z¹ is a difunctional moiety selected from ether, ester, amide, anhydride, urethane, carbamate or carbonate.

3. An implantable medical device comprising an aromatic polyanhydride, wherein the polyanhydride comprises at least one repeating unit of the chemical formula 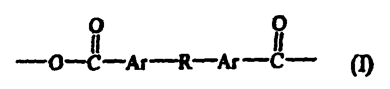 wherein
Ar is a substituted or unsubstituted aromatic ring and
R is - Z¹ - R¹ - Z¹*-* substituted ortho to the anhydride group on Ar, wherein R¹ is a difunctional organic moiety and Z¹ is a difunctional moiety selected from ester, amide, anhydride, urethane, carbamate or carbonate, and wherein the polyanhydride hydrolyzes to form a pharmaceutically active compound.

4. A medical device of claim 3, wherein R¹ is (-CH₂-)ₙ, (-CH₂CH₂-O-)ₘ, (-CH₂-CH₂-CH₂-O-)ₘ or (-CH₂CHCH₃-O-)ₘ wherein n is from 1 to 20, inclusive and m is selected so that R₁ has between 2 and 20 carbon atoms, inclusive.

5. A polyanhydride or medical device recording to any one of claim 1 to 4, wherein R¹ is -R²-Z²-R³-, wherein
R² and R³ are difunctional organic moieties selected, independently from one another, from (C₁-C₁₉) alkylene, (-CH₂-CH₂-O-)ₘ, (-CH₂CH₂CH₂-O-)ₘ or (-CH₂CHCH₃-O-)ₘ, wherein m is between 2 and 18, inclusive, and
Z² is a difunctional moiety selected from ether, ester, anhydride, amide, methane, carbamate or carbonate.

6. A polyanhydride or medical device according to any one or claims 1 to 5, wherein the polyanhydride hydrolyzes to an active compound selected from non-steroidal anti-inflammatory drugs (NSAID) selected among naphthyl and phenyl propionates anti-fibrotic aminobenzoate compounds or vasoconstricting agents.

7. A polyanhydride or medical device according to any one of claim 1 to 6*,* wherein the polyanhydride hydrolyzes to form therapeutic salicylates, therapeutic non-sterroidal and-inflammatory naphthyl or phenyl propionates, or therapeutic aminobenzoates or phenylethanolamines.

8. A polyanhydride or medical device according to claim 7, wherein the therapeutic Is thymotic acid, 4-sulfanilamidosalicylic acid, salicylsulfuric acid, salsalate, acetyl salicylic acid, mesalamine, gentisic acid, enfenamic acid, cresotic acid, aminosalicylic acid or aminophenylacetic acid.

9. A polyanhydride or medical device according to claim 7, wherein the therapeutic salicylate is thymotic acid, 4-sulfanilamidosalicylic acid, salicylsulfuric acid, salsalate, salicylic acid, mesalamine, gentisic acid, cresotic acid or aminosalicylic acid.

10. A polyanhydride or medical device according to any one of claims 1 to 9, wherein the polyanhydride is biocompatible and biodegradable,

11. A polyanhydride or medical device according to any one of claims I to 10, further comprising a biologically or pharmaceutically active compound in combination with the polyanhydride.

12. A polyanhydride or medical device according to claim 11, wherein the active compound is admixed with, embedded in, or dispersed into, or covalently attached to the polyanhydride.

13. A medical device according to any one of claims 3 to 12 which is a vascular graft, stent, a bone plate, suture, sensor or drug delivery device.

14. A medical device of claim 13, wherein the stent is a vascular stent.

15. A medical device of claim 3, wherein the device is a bone plate.

16. A medical device of claim 3, wherein the device is a drug delivery device.

17. A polyanhydride or medical device according to any one of claims 1 to 16 wherein Ar is a phenyl group.

18. A polyanhydride or medical device according to claim 13, 14 or 17, wherein Z¹ is an ester group and R¹ is (C₁-C₂₀) alkylene and the polyanhydride hydrolyzes to form a biologically active salicylate.

19. A polyanhydride or medical device according to any one of claims 1 to 18 formulated as a film, a coating, a dish or a sponge.

20. A polyanhydride according to claim 1 for use in medical therapy, wherein the polyanhydride hydrolyzes to form a pharmaceutically active compound.

21. A polyanhydride according to claim 2 for use in medical therapy, wherein Z¹ is a difunctional moiety selected from ester, amide, anhydride, urethane, carbamate or carbonate and wherein the polyanhydride hydrolyzes to form a pharmaceutically active compound

22. Use of an aromatic polyanhydride comprising at least one repeating unit of the chemical formula 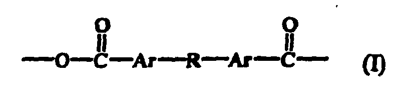 wherein
Ar is a substituted or unsubstituted aromatic ring and R is -Z¹-R¹-Z¹- substituted ortho to the anhydride group on Ar, wherein R¹ is a difunctional organic moiety a nd Z¹ is a difunctional moiety selected among ester, amide, anhydride, urethane, carbamate or carbonate, and wherein the polyanhydride hydrolyzes to form a pharmaceutically active compound, for the preparation of a medicament for the treatment of inflammation.

23. Use according to claim 22, wherein the inflammation is a digestive inflammation.

24. Use of an aromatic polyanhydride comprising at least one repeating unit of the chemical formula 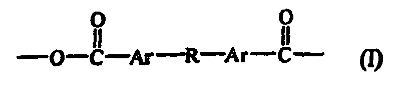 wherein
Ar is a substituted or unsubstituted aromatic ring and R is - Z¹- R¹- Z¹- substituted ortho to the anhydride group on Ar, wherein R¹ is a difunctional organic moiety and Z¹ is a difunctional moiety selected from ester, amide, anhydride, urethane, carbamate or carbonate, and wherein the polyanhydride hydrolyzes to form a pharmaceutically active compound, for the preparation of an implantable device, a drug delivery device, or a scaffolding implant for tissue reconstruction.

25. Use according to any one of claims 20 to 24 wherein R¹ is - R² - Z² - R³-, wherein
R² and R³ are difunctional organic moieties independently selected from (C₁-C₁₉) alkylene, (-CH₂-CH₂-O-)ₘ, (-CH₂-CH₂-CH₂-O-)ₘ or (-CH₂-CHCH₃-O-)ₘ, wherein m is between 2 and 18, inclusive, and
Z² is a difunctional moiety selected from ether, ester, anhydride, amide, urethane, carbamate or carbonate.

26. Use according to any of claims 20 to 25, wherein the polyanhydride hydrolyzes to form a biologically active non-steroidal anti-inflammatory drug (NSAID) selected among naphthyl and phenyl propionates, anti-fibrotic aminobenzoate compounds or vasoconstricting agent.

27. Use according to any of claims 20 to 26, wherein the polyanhydride hydrolyzes to form therapeutic salicylates, therapeutic non-steroidal anti-inflammatory naphthyl or phenyl propionates, or therapeutic aminobenzoates or phenylethanolamines.

28. Use according to claim 27, wherein the therapeutic is thymotic acid, 4-sulfanilamidosalicylic acid, salicylsulfuric acid, salsalate, acetyl salicylic acid, mesalamine, gentisic acid, enfenamic acid, cresotic acid, aminosalicylic acid or aminophenylacetic acid.

29. Use according to claim 27, wherein the therapeutic salicylate is thymotic acid, 4-sulfanilamidosalicylic acid, salicylsulfuric acid, salsalate, salicylic acid, mesalamine, gentisic acid, cresotic acid or aminosalicylic acid.

30. Use according to any of claims 20 to 29, wherein the polyanhydride is biocompatible and biodegradable.

31. Use according to any one of claims 20 to 30, wherein the polyanhydride further comprises at least one additional biologically or pharmaceutically active compound.

32. Use according to claim 31, wherein the additional active compound is admixed with, embedded in, dispersed or incorporated into, or covalently attached to the polyanhydride.

33. Use according to any one of claims 20 to 32*,* wherein the medicament or the implantable device is a vascular graft, stent, bone plate, suture, sensor or drug delivery device.

34. Use according to claim 33, wherein the stent is a vascular stent.

35. according to claim 20, wherein the device is a bone plate.

36. Use according to claim 20, wherein the implantable device is a drug delivery device.

37. Use according to any one of claims 20 to 36, wherein Ar is a phenyl group.

38. Use according to any one of claims 20 to 37, wherein the medicament or device is formulated as a film, a coating, a dish or a sponge.

39. Use according to any one of claims 33, 34 or 37, wherein Z¹ is an ester group and R¹ is (C₁-C₂₀) alkylene and the polyanhydride hydrolyzes to form a biologically active salicylate.

40. Use according to claim 27, wherein the polyanhydride hydrolyzes to form a therapeutic salicylate.

## Patentansprüche

1. Aromatisches Polyanhydrid, umfassend wenigstens eine Wiederholungseinheit der chemischen Formel 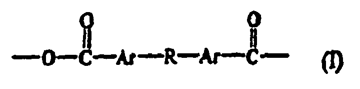 wobei
Ar ein substituierter oder unsubstituierter aromatischer Ring ist und
R -Z¹-R¹-Z¹-, substituiert ortho zu der Anhydridgruppe an Ar, ist, wobei R¹ eine difunktionelle organische Einheit ist und Z¹ eine difunktionelle Einheit, ausgewählt aus Amid, Anhydrid, Urethan, Carbamat oder Carbonat, ist.

2. Aromatisches Polyanhydrid, umfassend wenigstens eine Wiederholungseinheit der chemischen Formel 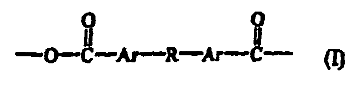 wobei
Ar ein substituierter oder unsubstituierter aromatischer Ring ist und
R -Z¹-R¹-Z¹-, substituiert ortho zu der Anhydridgruppe an Ar, ist, wobei R¹ eine Gruppe mit 2 bis 20 Kohlenstoffatomen mit einer Struktur, die ausgewählt ist aus (-CH₂CH₂-O-)ₘ, (-CH₂CH₂CH₂-O-)ₘ und (-CH₂CHCH₃-O-)ₘ, ist und Z¹ eine difunktionelle Einheit, ausgewählt aus Ether, Ester, Amid, Anhydrid, Urethan, Carbamat oder Carbonat, ist.

3. Implantierbare medizinische Vorrichtung, umfassend ein aromatisches Polyanhydrid, wobei das Polyanhydrid wenigstens eine Wiederholungseinheit der folgenden chemischen Formel umfasst: 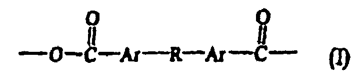 wobei
Ar ein substituierter oder unsubstituierter aromatischer Ring ist und
R -Z¹-R¹-Z¹-, substituiert ortho zu der Anhydridgruppe an Ar, ist, wobei R¹ eine difunktionelle organische Einheit ist und Z¹ eine difunktionelle Einheit, ausgewählt aus Ester, Amid, Anhydrid, Urethan, Carbamat oder Carbonat, ist und wobei das Polyanhydrid unter Bildung einer pharmazeutisch aktiven Verbindung hydrolysiert.

4. Medizinische Vorrichtung nach Anspruch 3, wobei R¹ (-CH₂-)ₙ, (-CH₂-CH₂-O-)ₘ, (-CH₂-CH₂-CH₂-O-)ₘ oder (-CH₂-CHCH₃-O-)ₘ ist, wobei n 1 bis 20, einschließlich, ist und m so ausgewählt ist, dass R¹ zwischen 2 und 20 Kohlenstoffatome, einschließlich, aufweist.

5. Polyanhydrid oder medizinische Vorrichtung nach einem der Ansprüche 1 bis 4, wobei R¹ -R²-Z²-R³- ist, wobei
R² und R³ difunktionelle organische Einheiten, unabhängig voneinander ausgewählt aus (C₁-C₁₉)-Alkylen, (-CH₂-CH₂-O-)ₘ, (-CH₂-CH₂-CH₂-O-)ₘ oder (-CH₂-CHCH₃-O-)ₘ, wobei m zwischen 2 und 18, einschließlich, ist, sind und
Z² eine difunktionelle Einheit, ausgewählt aus Ether, Ester, Anhydrid, Amid, Urethan, Carbamat oder Carbonat, ist.

6. Polyanhydrid oder medizinische Vorrichtung nach einem der Ansprüche 1 bis 5, wobei das Polyanhydrid zu einer aktiven Verbindung, ausgewählt aus nicht-steroidalen entzündungshemmenden Arzneimitteln (NSAID), ausgewählt aus Naphthyl- und Phenylpropionaten, antifibrotischen Aminobenzoatverbindungen oder gefäßverengenden Mitteln, hydrolysiert.

7. Polyanhydrid oder medizinische Vorrichtung nach einem der Ansprüche 1 bis 6, wobei das Polyanhydrid unter Bildung von therapeutischen Salicylaten, therapeutischen nicht-steroidalen entzündungshemmenden Naphthyl- oder Phenylpropionaten oder therapeutischen Aminobenzoaten oder Phenylethanolaminen hydrolysiert.

8. Polyanhydrid oder medizinische Vorrichtung nach Anspruch 7, wobei das Therapeutikum Thymotinsäure, 4-Sulfanilamidosalicylsäure, Salicylschwefelsäure, Salsalat, Acetylsalicylsäure, Mesalamin, Gentisinsäure, Enfenaminsäure, Cresotinsäure, Aminosalicylsäure oder Aminophenylessigsäure ist.

9. Polyanhydrid oder medizinische Vorrichtung nach Anspruch 7, wobei das therapeutische Salicylat Thymotinsäure, 4-Sulfanilamidosalicylsäure, Salicylschwefelsäure, Salsalat, Salicylsäure, Mesalamin, Gentisinsäure, Cresotinsäure oder Aminosalicylsäure ist.

10. Polyanhydrid oder medizinische Vorrichtung nach einem der Ansprüche 1 bis 9, wobei das Polyanhydrid biokompatibel und biologisch abbaubar ist.

11. Polyanhydrid oder medizinische Vorrichtung nach einem der Ansprüche 1 bis 10, außerdem umfassend eine biologisch oder pharmazeutisch aktive Verbindung in Kombination mit dem Polyanhydrid.

12. Polyanhydrid oder medizinische Vorrichtung nach Anspruch 11, wobei die aktive Verbindung mit dem Polyanhydrid vermischt, darin eingebettet oder dispergiert oder kovalent daran gebunden ist.

13. Medizinische Vorrichtung nach einem der Ansprüche 3 bis 12, welche ein Gefäßtransplantat, ein Stent, eine Knochenplatte, eine Naht, ein Sensor oder eine Arzneimittelabgabevorrichtung ist.

14. Medizinische Vorrichtung nach Anspruch 13, wobei der Stent ein Gefäßstent ist.

15. Medizinische Vorrichtung nach Anspruch 3, wobei die Vorrichtung eine Knochenplatte ist.

16. Medizinische Vorrichtung nach Anspruch 3, wobei die Vorrichtung eine Arzneimittelabgabevorrichtung ist.

17. Polyanhydrid oder medizinische Vorrichtung nach einem der Ansprüche 1 bis 16, wobei Ar eine Phenylgruppe ist.

18. Polyanhydrid oder medizinische Vorrichtung nach Anspruch 13, 14 oder 17, wobei Z¹ eine Estergruppe ist und R¹ (C₁-C₂₀)-Alkylen ist und das Polyanhydrid unter Bildung eines biologisch aktiven Salicylats hydrolysiert.

19. Polyanhydrid oder medizinische Vorrichtung nach einem der Ansprüche 1 bis 18, formuliert als ein Film, ein Überzug, eine Schüssel bzw. Platte oder ein Schwamm.

20. Polyanhydrid nach Anspruch 1 zur Verwendung in einer medizinischen Therapie, wobei das Polyanhydrid unter Bildung einer pharmazeutisch aktiven Verbindung hydrolysiert.

21. Polyanhydrid nach Anspruch 2 zur Verwendung in einer medizinischen Therapie, wobei Z¹ eine difunktionelle Einheit, ausgewählt aus Ester, Amid, Anhydrid, Urethan, Carbamat oder Carbonat, ist und wobei das Polyanhydrid unter Bildung einer pharmazeutisch aktiven Verbindung hydrolysiert.

22. Verwendung eines aromatischen Polyanhydrids, umfassend wenigstens eine Wiederholungseinheit der chemischen Formel 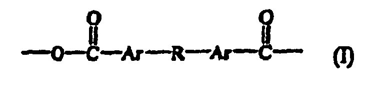 wobei
Ar ein substituierter oder unsubstituierter aromatischer Ring ist und R
-Z¹-R¹-Z¹-, substituiert ortho zu der Anhydridgruppe an Ar, ist, wobei R¹ eine difunktionelle organische Einheit ist und Z¹ eine difunktionelle Einheit, ausgewählt aus Ester, Amid, Anhydrid, Urethan, Carbamat oder Carbonat, ist und wobei das Polyanhydrid unter Bildung einer pharmazeutisch aktiven Verbindung hydrolysiert, zur Herstellung eines Medikaments für die Behandlung einer Entzündung.

23. Verwendung nach Anspruch 22, wobei die Entzündung eine Entzündung des Verdauungstrakts (digestive inflammation) ist.

24. Verwendung eines aromatischen Polyanhydrids, umfassend wenigstens eine Wiederholungseinheit der chemischen Formel 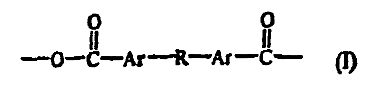 wobei
Ar ein substituierter oder unsubstituierter aromatischer Ring ist und R
-Z¹-R¹-Z¹-, substituiert ortho zu der Anhydridgruppe an Ar, ist, wobei R¹ eine difunktionelle organische Einheit ist und Z¹ eine difunktionelle Einheit, ausgewählt aus Ester, Amid, Anhydrid, Urethan, Carbamat oder Carbonat, ist und wobei das Polyanhydrid unter Bildung einer pharmazeutisch aktiven Verbindung hydrolysiert, zur Herstellung einer implantierbaren Vorrichtung, einer Arzneimittelabgabevorrichtung oder eines Gerüstimplantats zur Geweberekonstruktion.

25. Verwendung nach einem der Ansprüche 20 bis 24, wobei R¹ -R²-Z²-R³- ist, wobei
R² und R³ difunktionelle organische Einheiten sind, die unabhängig voneinander ausgewählt sind aus (C₁-C₁₉)-Alkylen, (-CH₂-CH₂-O-)ₘ, (-CH₂-CH₂-CH₂-O-)ₘ oder (-CH₂-CHCH₃-O-)ₘ, wobei m zwischen 2 und 18, einschließlich, ist, und
Z² eine difunktionelle Einheit, ausgewählt aus Ether, Ester, Anhydrid, Amid, Urethan, Carbamat oder Carbonat, ist.

26. Verwendung nach einem der Ansprüche 20 bis 25, wobei das Polyanhydrid unter Bildung eines biologisch aktiven nicht-steroidalen entzündungshemmenden Arzneimittels (NSAID), ausgewählt aus Naphthyl- und Phenylpropionaten, von antifibrotischen Aminobenzoatverbindungen oder eines gefäßverengenden Mittels hydrolysiert.

27. Verwendung nach einem der Ansprüche 20 bis 26, wobei das Polyanhydrid unter Bildung von therapeutischen Salicylaten, therapeutischen nicht-steroidalen entzündungshemmenden Naphthyl- oder Phenylpropionaten oder therapeutischen Aminobenzoaten oder Phenylethanolaminen hydrolysiert.

28. Verwendung nach Anspruch 27, wobei das Therapeutikum Thymotinsäure, 4-Sulfanilamidosalicylsäure, Salicylschwefelsäure, Salsalat, Acetylsalicylsäure, Mesalamin, Gentisinsäure, Enfenaminsäure, Cresotinsäure, Aminosalicylsäure oder Aminophenylessigsäure ist.

29. Verwendung nach Anspruch 27, wobei das therapeutische Salicylat Thymotinsäure, 4-Sulfanilamidosalicylsäure, Salicylschwefelsäure, Salsalat, Salicylsäure, Mesalamin, Gentisinsäure, Cresotinsäure oder Aminosalicylsäure ist.

30. Verwendung nach einem der Ansprüche 20 bis 29, wobei das Polyanhydrid biokompatibel und biologisch abbaubar ist.

31. Verwendung nach einem der Ansprüche 20 bis 30, wobei das Polyanhydrid au-ßerdem wenigstens eine zusätzliche biologisch oder pharmazeutisch aktive Verbindung umfasst.

32. Verwendung nach Anspruch 31, wobei die zusätzliche aktive Verbindung mit dem Polyanhydrid vermischt, darin eingebettet, dispergiert oder eingearbeitet oder kovalent daran gebunden ist.

33. Verwendung nach einem der Ansprüche 20 bis 32, wobei das Medikament oder die implantierbare Vorrichtung ein Gefäßtransplantat, ein Stent, eine Knochenplatte, eine Naht, ein Sensor oder eine Arzneimittelabgabevorrichtung ist.

34. Verwendung nach Anspruch 33, wobei der Stent ein Gefäßstent ist.

35. Verwendung nach Anspruch 20, wobei die Vorrichtung eine Knochenplatte ist.

36. Verwendung nach Anspruch 20, wobei die implantierbare Vorrichtung eine Arzneimittelabgabevorrichtung ist.

37. Verwendung nach einem der Ansprüche 20 bis 36, wobei Ar eine Phenylgruppe ist.

38. Verwendung nach einem der Ansprüche 20 bis 37, wobei das Medikament oder die Vorrichtung als ein Film, ein Überzug, eine Schüssel bzw. Platte oder ein Schwamm formuliert ist.

39. Verwendung nach einem der Ansprüche 33, 34 oder 37, wobei Z¹ eine Estergruppe ist und R¹ (C₁-C₂₀)-Alkylen ist und das Polyanhydrid unter Bildung eines biologisch aktiven Salicylats hydrolysiert.

40. Verwendung nach Anspruch 27, wobei das Polyanhydrid unter Bildung eines therapeutischen Salicylats hydrolysiert.

## Revendications

1. Polyanhydride aromatique comprenant au moins un motif répétitif de formule chimique 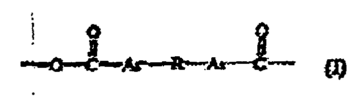 dans laquelle
Ar est un anneau aromatique substitué ou non substitué et
R est -Z¹-R¹-Z¹-substitué en ortho au groupement anhydride sur Ar, dans laquelle R¹ est une fraction organique difonctionnelle et Z¹ est une fraction difonctionnelle choisie parmi des amides, anhydrides, uréthanes, carbamates ou carbonates.

2. Polyanhydride aromatique comprenant au moins un motif répétitif de formule chimique 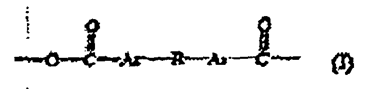 dans laquelle
Ar est un anneau aromatique substitué ou non substitué et
R est -Z¹-R¹-Z¹-substitué en ortho au groupement anhydride sur Ar, dans laquelle R¹ est un groupement comportant 2 à 20 atomes de carbone ayant une structure choisie parmi (-CH₂CH₂-O-)ₘ,
(-CH₂CH₂CH₂-O-)ₘ et (-CH₂CHCH₃-O-)ₘ et Z¹ est une fraction difonctionnelle choisie parmi des éthers, esters, amides, anhydrides, uréthanes, carbamates ou carbonates.

3. Dispositif médical implantable comprenant un polyanhydride aromatique, dans lequel le polyanhydride comprend au moins un motif répétitif de formule chimique 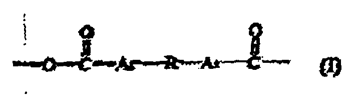 dans laquelle
Ar est un anneau aromatique substitué ou non substitué et
R est -Z¹-R¹-Z¹-substitué en ortho au groupement anhydride sur Ar, dans lequel R¹ est une fraction organique difonctionnelle et Z¹ est une fraction difonctionnelle choisie parmi des esters, amides, anhydrides, uréthanes, carbamates ou carbonates, et dans lequel le polyanhydride s'hydrolyse pour former un composé pharmaceutiquement actif.

4. Dispositif médical selon la revendication 3, dans lequel R¹ est (-CH₂-)ₘ (-CH₂-CH₂-O-)ₘ, (-CH₂-CH₂-CH₂-O-)ₘ ou (-CH₂-CHCH₃-O-)ₘ dans lequel n est de 1 à 20, inclus, et m est choisi de sorte que R₁ a entre 2 et 20 atomes de carbone, inclus.

5. Polyanhydride ou dispositif médical selon l'une quelconque des revendications 1 à 4, dans lequel R¹ est -R²-Z²-R³-, dans lequel
R² et R³ sont des fractions organiques difonctionnelles, choisies indépendamment l'un de l'autre, parmi un alkylène en C₁ à C₁₉, (-CH₂-CH₂-O-)ₘ, (-CH₂-CH₂-CH₂-O-), ou (-CH₂-CHCH₃-O-)ₘ dans lequel m est entre 2 et 18, inclus, et
Z² est une fraction difonctionnelle choisie dans le groupe comprenant des éthers, esters, anhydrides, amides, uréthanes, carbamates ou carbonates.

6. Polyanhydride ou dispositif médical selon l'une quelconque des revendications 1 à 5, dans lequel le polyanhydride s'hydrolyse en composé actif choisi parmi des médicaments anti-inflammatoires non stéroïdiens (AINS) choisis parmi des propionates de naphtyle et de phényle, des composés aminobenzoates antifibrotiques ou des agents vasoconstricteurs.

7. Polyanhydride ou dispositif médical selon l'une quelconque des revendications 1 à 6, dans lequel le polyanhydride hydrolyse pour former des salicylates thérapeutiques, des propionates de naphtyle ou de phényle anti-inflammatoires non stéroïdiens thérapeutiques, ou des aminobenzoates thérapeutiques ou des phényléthanolamines.

8. Polyanhydride ou dispositif médical selon la revendication 7, dans lequel l'agent thérapeutique est l'acide thymotique, acide 4-sulfanilamidosalicylique, acide salicylsulfurique, le salsalate, l'acide acétylsalicylique, la mésalamine, l'acide gentisique, l'acide enfénamique, l'acide crésotique, l'acide aminosalicylique ou l'acide aminophénylacétique.

9. Polyanhydride ou dispositif médical selon la revendication 7, dans lequel le salicylate thérapeutique est l'acide thymotique, l'acide 4-sulfanilamidosalicylique, l'acide salicylsulfurique, le salsalate, l'acide salicylique, la mésalamine, l'acide gentisique, l'acide crésotique ou l'acide aminosalicylique.

10. Polyanhydride ou dispositif médical selon l'une quelconque des revendications 1 à 9, dans lequel le polyanhydride est biocompatible et biodégradable.

11. Polyanhydride ou dispositif médical selon l'une quelconque des revendications 1 à 10, comprenant en outre un composé biologiquement ou pharmaceutiquement actif en association avec le polyanhydride.

12. Polyanhydride ou dispositif médical selon la revendication 11, dans lequel le composé actif est mélangé avec, imprégné dans, ou dispersé dans, ou attaché de manière covalente au polyanhydride.

13. Dispositif médical selon l'une quelconque des revendications 3 à 12 qui est une greffe vasculaire, une endoprothèse, une plaque d'ostéosynthèse, une suture, un détecteur ou un dispositif d'administration de médicament.

14. Dispositif médical selon la revendication 13, dans lequel l'endoprothèse est une endoprothèse vasculaire.

15. Dispositif médical selon la revendication 3, dans lequel le dispositif est une plaque d'ostéosynthèse.

16. Dispositif médical selon la revendication 3, dans lequel le dispositif est un dispositif d'administration de médicament.

17. Polyanhydride ou dispositif médical selon l'une quelconque des revendications 1 à 16, dans lequel Ar est un groupement phényle.

18. Polyanhydride ou dispositif médical selon la revendication 13, 14 ou 17, dans lequel Z¹ est un groupement ester et R¹ est un alkylène en C₁ à C₂₀ et le polyanhydride s'hydrolyse pour former un salicylate biologiquement actif.

19. Polyanhydride ou dispositif médical selon l'une quelconque des revendications 1 à 18 présenté sous la forme d'un film, d'un revêtement ou d'une éponge.

20. Polyanhydride selon la revendication 1, pour une utilisation dans une thérapie médicale, dans lequel le polyanhydride hydrolyse pour former un composé pharmaceutiquement actif.

21. Polyanhydride selon la revendication 2, pour une utilisation dans une thérapie médicale, dans lequel Z¹ est une fraction difonctionnelle choisie parmi des esters, amides, anhydrides, uréthanes, carbamates ou carbonates et dans lequel le polyanhydride hydrolyse pour former un composé pharmaceutiquement actif.

22. Utilisation d'un polyanhydride aromatique comprenant au moins un motif répétitif de formule chimique 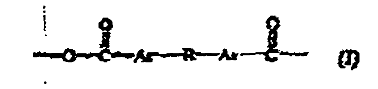 dans lequel
Ar est un anneau aromatique substitué ou non substitué et R est -Z¹-R¹-Z¹-substitué en ortho au groupement anhydride sur Ar, dans lequel R¹ est une fraction organique difonctionnelle et Z¹ est une fraction difonctionnelle choisie parmi des esters, amides, anhydrides, uréthanes, carbamates ou carbonates, et dans lequel le polyanhydride hydrolyse pour former un composé pharmaceutiquement actif, pour la préparation d'un médicament pour le traitement de l'inflammation.

23. Utilisation selon la revendication 22, dans laquelle l'inflammation est une inflammation digestive.

24. Utilisation d'un polyanhydride aromatique comprenant au moins un motif répétitif de formule chimique 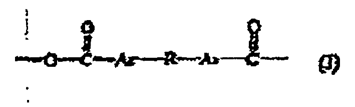 dans lequel
Ar est un anneau aromatique substitué ou non substitué et R est -Z¹-R¹-Z¹-substitué en ortho au groupement anhydride sur Ar, dans lequel R¹ est une fraction organique difonctionnelle et Z¹ est une fraction difonctionnelle choisie parmi des esters, amides, anhydrides, uréthanes, carbamates ou carbonate, et dans lequel le polyanhydride hydrolyse pour former un composé pharmaceutiquement actif, pour la préparation d'un dispositif implantable, un dispositif d'administration de médicament, ou un implant servant de support pour la reconstruction tissulaire.

25. Utilisation selon l'une quelconque des revendications 20 à 24, dans laquelle R¹ est -R²-Z-²R³-, dans lequel
R² et R³ sont des fractions organiques difonctionnelles, choisies indépendamment parmi des alkylènes en C₁ à C₁₉, (-CH₂-CH₂-O-)ₘ, (-CH₂-CH₂-CH₂-O-)ₘ ou (-CH₂-CHCH₃-O-)ₘ dans lequel m est entre 2 et 18, inclus, et
Z² est une fraction difonctionnelle choisie parmi des éthers, esters, anhydrides, amides, uréthanes, carbamates ou carbonates.

26. Utilisation selon l'une quelconque des revendications 20 à 25, dans laquelle le polyanhydride hydrolyse pour former un médicament anti-inflammatoire non stéroïdien (AINS) biologiquement actif choisi parmi des propionates de naphtyle et de phényle, des agents aminobenzoates antifibrotiques ou des agents vasoconstricteurs.

27. Utilisation selon l'une quelconque des revendications 20 à 26, dans laquelle le polyanhydride hydrolyse pour former des salicylates thérapeutiques, des propionates de naphtyle ou de phényle anti-inflammatoires non stéroïdiens thérapeutiques, ou des aminobenzoates thérapeutiques ou des phényléthanolamines.

28. Utilisation selon la revendication 27, dans laquelle l'agent thérapeutique est l'acide thymotique, l'acide 4-sulfanilamidosalicylique, l'acide salicylsulfurique, le salsalate, l'acide acétylsalicylique, la mésalamine, l'acide gentisique, l'acide enfénamique, l'acide crésotique, l'acide aminosalicylique ou l'acide aminophénylacétique.

29. Utilisation selon la revendication 27, dans laquelle le salicylate thérapeutique est l'acide thymotique, l'acide 4-sulfanilamidosalicylique, l'acide salicylsulfurique, le salsalate, l'acide acétylsalicylique, la mésalamine, l'acide gentisique, l'acide crésotique ou l'acide aminosalicylique.

30. Utilisation selon l'une quelconque des revendications 20 à 29, dans laquelle le polyanhydride est biocompatible et biodégradable.

31. Utilisation selon l'une quelconque des revendications 20 à 30, dans laquelle le polyanhydride comprend en outre au moins un composé biologiquement ou pharmaceutiquement actif.

32. Utilisation selon la revendication 31, dans laquelle le composé actif supplémentaire est mélangé avec, imprégné dans, dispersé ou incorporé dans, ou attaché de manière covalente au polyanhydride.

33. Utilisation selon l'une quelconque des revendications 20 à 32, dans laquelle le médicament ou le dispositif implantable est une greffe vasculaire, une endoprothèse, une plaque d'ostéosynthèse, une suture, un détecteur ou un dispositif d'administration de médicament.

34. Utilisation selon la revendication 33, dans laquelle l'endoprothèse est une endoprothèse vasculaire.

35. Utilisation selon la revendication 20, dans laquelle le dispositif est une plaque d'ostéosynthèse.

36. Utilisation selon la revendication 20, dans laquelle le dispositif implantable est un dispositif d'administration de médicament.

37. Utilisation selon l'une quelconque des revendications 20 à 36, dans laquelle Ar est un groupement phényle.

38. Utilisation selon l'une quelconque des revendications 20 à 37, dans laquelle le médicament ou dispositif est présenté comme un film, un revêtement ou une éponge.

39. Utilisation selon l'une quelconque des revendications 33, 34 ou 37, dans laquelle Z¹ est un groupement ester et R¹ est un alkylène en C₁ à C₂₀ et le polyanhydride hydrolyse pour former un salicylate biologiquement actif.

40. Utilisation selon la revendication 27, dans laquelle le polyanhydride hydrolyse pour former un salicylate thérapeutique.
